# EUROPÄISCHE PATENTSCHRIFT

(11) **EP 1 848 675 B1**
(45) Veröffentlichungstag und Bekanntmachung des Hinweises auf die Patenterteilung: **03.08.2011**
(21) Anmeldenummer: 05798809.9
(22) Anmeldetag: 04.11.2005
(51) Int. Cl.: C05F 7/00, B01F 7/04, B01F 15/00, C05F 17/00, C12M 1/06

(54) **FERMENTER MIT RÜHRWERK**
FERMENTER COMPRISING AN AGITATOR
FERMENTEUR DOTE D'UN MELANGEUR

(30) Priorität: 26.01.2005 CH 122052005
(43) Veröffentlichungstag der Anmeldung: 31.10.2007
(73) Patentinhaber: Kompogas AG, 8152 Glattbrugg (CH)
(72) Erfinder: SCHMID, Walter, 8152 Glattbrugg (CH)
(74) Vertreter: Schneider Feldmann AG Patent- und Markenanwälte
(86) Internationale Anmeldenummer: PCT/CH2005/000646
(87) Internationale Veröffentlichungsnummer: WO 2006/079227

(56) Entgegenhaltungen:
- EP-A- 0 324 894
- EP-A- 1 332 805
- DE-A- 3 149 344
- DE-A- 19 648 875
- US-A- 2 306 960
- US-A- 3 367 126
- US-A- 4 514 297

## Beschreibung

Die vorliegende Erfindung betrifft einen pfropfstrombetriebenen Fermenter zur anaeroben Fermentation biogener Abfälle mit einem Einlass und einem Auslass und einem Rührwerk, welches aus einer den Fermenter in seiner Längsrichtung durchquerenden Welle besteht, an der eine Vielzahl von Rührarmen angeordnet sind, wobei die Welle im Bereich der Fermenterenden gelagert ist.

, Der prinzipielle Aufbau von Anlagen zur Erzeugung von Biogas aus biogenen Abfällen geht aus der Europäischen Patentschrift EP 0476217A hervor. Die Anlagen arbeiten nach einem Verfahren gemäss dem europäischen Patent EP-B-621 336. Das erstgenannte Schutzrecht offenbart einen pfropfstrombetriebenen liegenden Fermenter, der zur anaeroben Fermentation biogener Abfälle geeignet ist. Der Fermenter ist ein langgestreckter, liegender Tank mit einem an einem Ende vorgesehenen Einlass und einem am gegenüber liegenden Ende vorhandenen Auslass. Die biogenen Abfälle werden einlassseitig zerkleinert eingegeben und mit fermentiertem Gut und/oder Presswasser aus der Aufbereitung geimpft. Hierdurch wird das zu fermentierende Gut mit Methanbakterien angereichert. Im Fermenter werden nun unter kontrollierter Durchmischung die biogenen Abfälle unter Bildung von Biogas abgebaut und anschliessend nach dem Verlassen durch den Auslass einer aeroben Verrottung zugeführt. zur Färderung einer gleichförmigen Bewegung schlägt die EP-1332 805 A die Anordung der Rührarme spiral forming vor.

Die weltweite Nachfrage nach Anlagen der eingangs genannten Art mit immer grösseren Kapazitäten führt dazu, dass auch immer grössere Fermenter gebaut werden. Um dies zu ermöglichen, müssen die Fermentertanks vor Ort errichtet werden, wobei dies entweder durch die segmentweise Zusammenfügung zu einem Stahltank erfolgen kann oder, wie aus der EP-770 / 675-A bekannt ist, kann der liegende Fermentertank vor Ort aus Beton erstellt werden. Zur Erhöhung der Kapazität werden heute liegende Fermentertanks mit einer Gesamtlänge von über 50 Metern und einem Durchmesser von über 10 Metern realisiert. Bei den bisherigen Lösungen wurde die Welle des Rührwerkes als massive Stahlwelle gestaltet. Bei Längen unter 20 Metern ist dies auch relativ problemlos möglich. Werden grössere Längen erforderlich, so führt das Eigengewicht der Wellen zu einer Durchbiegung, die problematisch ist. Das Rührwerk muss nämlich nicht nur die biogenen Abfälle durchmischen um eine gewisse Homogenität zu erreichen, sondern gleichzeitig muss sichergestellt werden, dass schwere Festgüter, wie insbesondere Sand und Steine nicht am Boden des Fermentertanks sedimentieren und folglich nicht mehr ausgetragen werden. Obwohl der Fermenter im Pfropfstrom betrieben wird, vermag die Durchströmung die absinkenden Schwerstoffe nicht auszutragen, da die Pfropfstrombewegung nur eine geringe Strömungsgeschwindigkeit aufweist. Die Durchsatzzeit der biogenen Abfälle durch den Fermenter vom Einlass zum Auslass beträgt nämlich mehrere Tage. Das Rührwerk trägt folglich neben der Durchmischung ebenfalls dazu bei, diese Schwerstoffe vom Boden wieder nach oben zu befördern, um danach bei der nachfolgenden Sinkbewegung im Pfropfstrom Richtung Fermenterauslass transportiert zu werden. Entsprechend besteht das Rührwerk aus einer den Fermenter durchsetzenden Welle mit einer Vielzahl von Rührarmen, die an ihrem von der Welle abgelegenen Ende mit entsprechenden Schaufeln versehen sind.

Bei gross dimensionierten Fermentern hat nun die Durchbiegung der Welle dazu geführt, dass die Schaufeln praktisch an der Fermenterwand entlang streifen und entsprechend sind nach wenigen Betriebsjahren Defekte am Fermenter aufgetreten. In einer nächstliegenden Lösung wurde nach Feststellung des Problems die Welle mittels Zwischenstützen gelagert. Diese Lösung hat sich jedoch nicht bewährt, da die Stützen den Pfropfstrombetrieb empfindlich stören. ,

Schliesslich ist aus der DE-A-31 / 49 / 344 ein Fermenter bekannt, der ein Rührwerk aufweist, welches mit tankartigen Rührarmen versehen ist. Diese Rührarme sind so gestaltet, dass in diese Gas unter Verdrängung von Flüssiganteilen aus diesen tankartigen Rühramen einpumpbar ist, so dass deren Auftrieb eine Drehbewegung des Rührwerkes bewirkt, ohne dass eine getriebene Welle erforderlich ist.

In DE-A 31 49 344 ist eine Hohlwelle vorhanden die auf der ganzen Länge auf einer feststehenden Hohlachse lagert.

Auch der Fermenter gemäss der US-4 514 297 zeigt einen liegenden Tank mit einem eingangsseitigen Einlass und am anderen Ende einen Auslass. Zusätzlich kann in diesen Fermenter heisser Schlamm an jeder Stelle des Fermenters zugeführt werden. Hierzu ist in der Hohlwelle ein Schlitten angebracht, der mit Schlitzen in der Welle in kommunizierende Verbindung bringbar ist um den heissen Schlamm in den Fermenter zu induzieren.

Ebenfalls besitzt der Fermenter gemäss der DE- 19 48 875 eine Hohlwelle. Hier wird die Hohlwelle dazu verwendet eine zentrale Heizwasser-Zufuhr darin unterzubringen, von der aus entlang den Rührarmen Heizrohre verlaufen.

Es ist daher die Aufgabe der Erfindung eine Lösung anzubieten, die ein Durchbiegen der Welle vermeidet und die daraus folgenden Schäden ausschließt.

Diese Aufgabe löst ein pfropfstrombetriebener, liegender Fermenter gemäss Oberbegriff des Patentanspruches 1 mit den Merkmalen des kennzeichnenden Teils des Patentanspruches 1.

Weitere vorteilhafte Ausgestaltungsformen des Erfindungsgegenstandes gehen aus den abhängigen Ansprüchen hervor. Deren Ausgestaltung, Zweck und Wirkung ist in der nachfolgenden Beschreibung unter Bezug auf die anliegenden Zeichnungen erläutert. Es zeigt:
- Figur 1: einen vertikalen Längsschnitt durch einen erfindungsgemässen Fermenter und
- Figur 2: einen vertikalen Schnitt durch den Fermenter quer zur Wellenlängsrichtung.
- Figur 3: zeigt einen schematischen Querschnitt durch die Hohlwelle im Bereich eines Sensors und
- Figur 4: eine drahtlose Sensorüberwachungseinheit in schematischer Darstellung.
- Figur 5: zeigt eine herkömmliche Drucküberwachung durch den Wellenzapfen der erfindungsgemässen Hohlwelle.

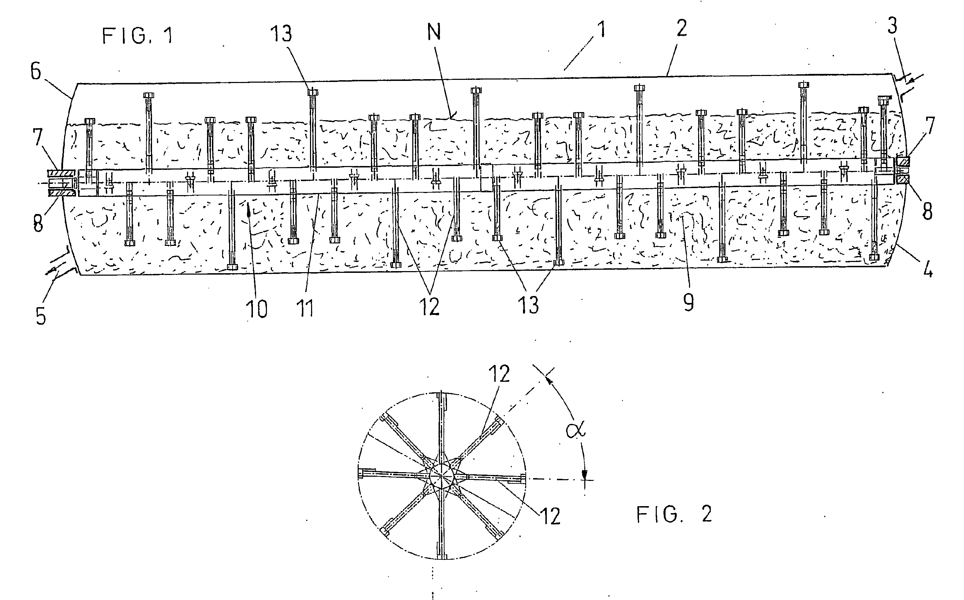

In der Figur 1 ist der erfindungsgemässe liegende Fermenter in seiner Gesamtheit in einem vertikalen Längsschnitt dargestellt. Der gesamte Fermenter ist mit 1 bezeichnet. Dieser umfasst den Fermentertank 2, der aus Stahl oder Beton gefertigt sein kann. Auf der einen Seite ist ein Einlass 3 in der einlassseitigen Stirnwand 4 vorhanden. Auf der gegenüber liegenden Seite ist ein Auslass 5 in der auslassseitigen Stirnwand 6 vorhanden. In beiden Stirnwänden 4 und 6 ist je ein Wellenlager 7 angeformt, in dem die Welle 10 mit ihren endständigen Wellenzapfen 8 lagert.

Die Welle 10 umfasst die beiden Wellenzapfen 8, die drehfest mit einem Wellenkörper 11 verbunden sind. Der Wellenkörper 11 besteht aus einem Stahlrohr, welches beidseitig hermetisch verschlossen ist. Am Wellenkörper 11 sind eine Vielzahl von Rührarmen 12 mittels einer entsprechenden Schweisskonstruktion angebracht. Jeder Rührarm 12 weist endständige Schaufeln 13 auf.

Eine bevorzugte Lösung besteht darin, dass das den Wellenkörper 11 bildende Rohr mit ein- oder beidseitig angeformten Wellenzapfen 8 versehen ist. Hierbei erfolgt der Antrieb ein- oder beidseitig über einen oder beide Wellenzapfen. Eine Variante besteht darin, dass man das den Wellenkörper 11 bildende Rohr ein- oder beidseitig durch die Stirnwände 4,6 des Fermenters hindurchführt und den Antrieb ein- oder beidseitig beispielsweise über einen auf dem Rohr angebrachten Zahnkranz zu realisieren.

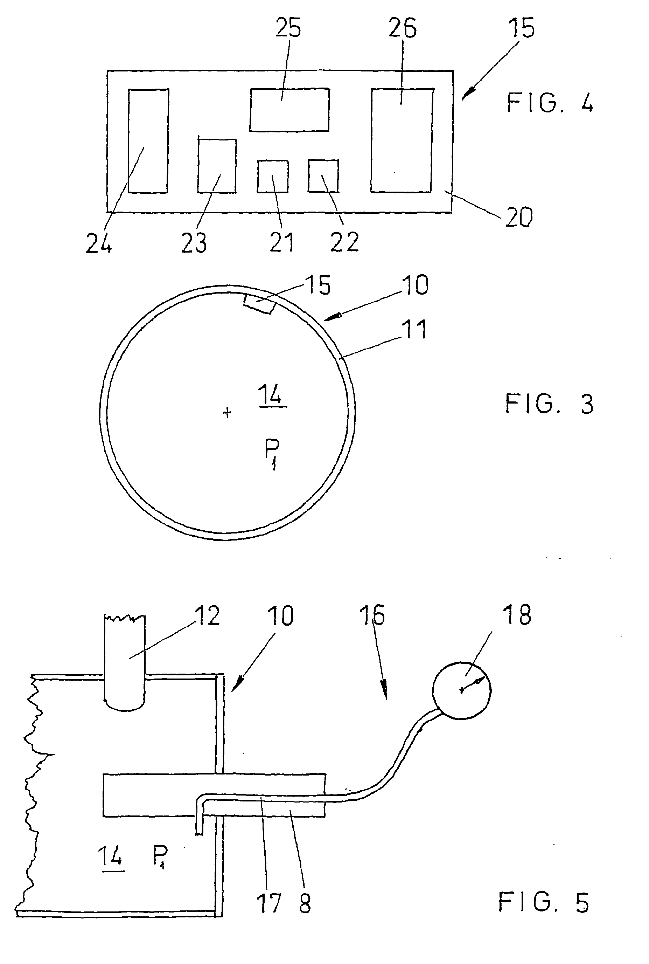

Die Welle 10 ist folglich als Hohlwelle ausgestaltet. In der Schnittzeichnung nach Figur 3 erkennt man den Wellenkörper 11 und dessen Innenraum 14. Auf der Innenwand des aus einem Rohr gebildeten Wellenkörpers 11 ist gemäss der hier dargestellten Ausführungsform eine Sensorüberwachungseinheit 15 angebracht. Im Innenraum 14 des Wellenkörpers 11 herrscht ein vorbestimmter, angelegter Druck. Dieser Druck ist mit P₁ bezeichnet. Der Druck P₁ im Innenraum 14 kann ein Überdruck oder ein Unterdruck sein. Die Überwachung des Innendruckes P₁ mittels der Sensorüberwachungseinheit 15 kann herkömmlich über entsprechende elektrische Leitungen, die im Bereich des Wellenzapfens nach aussen geführt werden, erfolgen oder, wie hier dargestellt und später mit Bezug auf die Figur 4 noch zu erläutern ist, durch drahtlose Übermittlung erfolgen.

Wie in der Figur 5 gezeigt, kann jedoch die Überwachung des Innendruckes P₁ auch herkömmlich erfolgen, indem eine Drucküberwachungseinheit 16 vorgesehen ist, die aus einer Druckleitung 17 und einem daran angeschlossenen Manometer 18 besteht. Der Druck P₁ im Innenraum 14 kann prinzipiell als Überdruck oder als Unterdruck gewählt werden. Dies ist insofern nicht von Bedeutung, da im Prinzip jede Form der Druckänderung im Innenraum 14 eine Indikation einer Leckage ist, die zu einer Abschaltung der Anlage und insbesondere zu einer Abschaltung des Rührwerkes führen muss.

Prinzipiell beruht die vorliegende Erfindung auf dem Gedanken, dass die Hohlwelle im Betriebszustand ständig und vollständig in der zu vergärenden Biomasse eingetaucht ist. Hierdurch verdrängt die Welle dank ihrer Gestaltung als Hohlwelle ein relativ grosses Volumen und entsprechend bewirkt der Innenraum 14 in der Welle 10 einen Auftrieb, der das Gewicht der Welle 10 beziehungsweise des Wellenkörpers 11 mit den daran befestigten Rührarmen 12 mit den Schaufeln 13 mindestens teilweise kompensiert. Da aber nach mehrjähriger Betriebsdauer die Wahrscheinlichkeit möglicher Undichtigkeiten durch Spannungsrisse oder mechanische Defekte zunimmt, und insbesondere auch Korrosionsschäden auftreten können, die zu Leckagen führen, dringt früher oder.später Feuchtigkeit in die Hohlwelle ein, die das Gesamtgewicht der Welle 10 verändern würde und entsprechend zu den bereits eingangs erwähnten Schäden führen würde. Da eine optische Kontrolle weder technisch vernünftig realisierbar ist noch die entsprechenden zum Teil haarrissgrossen Schäden erkennbar sein würden, geht die Erfindung von einer manometrischen Überwachung des Innenraumes 14 aus. Jeder Defekt der Welle, der zu einer Leckage führt, wird automatisch dazu führen, dass eine Druckveränderung im Innenraum 14 erfolgt. Der vorgegebene Druck P₁ wird sich folglich verändern. Ist der Innendruck P₁ als Unterdruck angelegt, so wird der Druck steigen, ist der Innendruck P₁ als Überdruck angelegt, so wird dieser Innendruck abfallen. In jedem Fall ist jedoch die Druckveränderung ein sicherer Indikator, dass eine Leckage vorhanden ist. Das Ausmass der Druckveränderung kann zudem einen Hinweis auf die Grösse des Schadens geben. Im Normalfall wird eine solche Druckveränderung dazu führen, dass die Anlage heruntergefahren wird, d.h. die Zufuhr der biogenen Abfälle wird abgestellt und der Fermenter bei Betriebsbedingungen weiterbetrieben und sukzessive entleert. Zur Lecksuche kann im Innenraum 14 ein starküberhöhter Druck angelegt werden, um damit das Ausströmen der Luft ermitteln zu können und so den Schadensort lokalisieren zu können. Es wird jedoch angenommen, dass solche Schadensereignisse relativ seltene Fälle sein werden.

Da der Füllgrad des Fermenters 1 meist bis auf ein Niveau N, welches weit über der Mitte des Fermenters liegt, erfolgt, ist die Welle 10 praktisch immer vollständig innerhalb des Bereiches der biogenen Masse, so dass auch immer der Auftrieb anliegt. Prinzipiell wäre es wünschenswert, wenn der Auftrieb der Welle 10 im mittigen Bereich der Welle bezogen auf die Längsausdehnung grösser wäre als an den Enden. An den Enden wird die Stützfunktion ohnehin durch die entsprechenden Wellenlager 7, in denen die Wellenzapfen 8 lagern, weitgehend aufgenommen. Um diese Möglichkeit zu realisieren, wäre es denkbar die Rührarme 12 im zentrischen Bereich er Welle 10 aus geschlossenen Rohren zu fertigen. Prinzipiell wäre es denkbar, dass diese Rohre der Rührarme 12 ebenfalls mit dem Innenraum 14 der Welle 10 kommunizieren.

Auf jeden Fall ist es erforderlich, dass die Rührarme bezüglich des Umfanges der Welle regelmässig verteilt angeordnet sind. Nicht zwingend erforderlich ist jedoch die gleichmässige Verteilung der Rührarme 12 über die Länge der Welle hinweg. Es ist daher durchaus möglich und sinnvoll, die Dichte der Rührarme im einlassseitigen und auslassseitigen Bereich zu erhöhen. Hierdurch wird die Sedimentation von Feststoffen insbesondere in diesen relativ sensiblen Bereichen verstärkt abgebaut. Die relative Freiheit der Anordnung der Rührarme und deren Ausgestaltung führt dazu, dass der Auftrieb der Welle insgesamt weitgehend austariert werden kann.

Die Verteilung der Rührarme am Umfang der Welle 10 soll gleichmässig sein. Bevorzugterweise beträgt der relative Winkel α zwischen zwei in Längsrichtung benachbarten Rührarmen 12 zwischen 90° und 30° und insbesondere bevorzugt beträgt der Winkel α 45°.

Der in der Figur 4 schematisch dargestellte Fertigbauteil 20 ist eine Sensorüberwachungseinheit 15, wie sie beispielsweise aus der Fahrzeugtechnik bekannt ist zur drahtlosen Überwachung des Reifendrucks, insbesondere bei Lastwagen. Hierzu kann beispielsweise auf die US-2004/0155764-A verwiesen werden.

Ein solches Fertigbauteil 20 besteht aus einer Basisplatte, auf der ein Drucksensor 21 angebracht ist. Zusätzlich aber durchaus nicht zwingend kann ein weiterer Sensor 22 vorgesehen sein, der als Temperatursensor oder Hygroskopsensor gestaltet sein kann. Ein Temperatursensor dient im Wesentlichen dazu, gewisse temperaturbedingte Druckschwankungen zu realisieren, damit diese nicht zu Fehlinterpretationen führen.

Eine Übertragungsantenne 24 kann hochfrequente Signale empfangen, die in einen Gleichstrom umgewandelt werden können und eine Speiseeinheit 26 bilden. Diese Energie speist dann einen Mikrocomputer 25, der die Daten der Sensoren 21,22 auswertet und ein Signal an einen Radiosender 23 liefert. Das gesendete Signal wird von einer Überwachung der gesamten Fermentationsanlage ausgewertet und kann gegebenenfalls zu einer Abschaltung des Rührwerks und/oder zum Herunterfahren der Anlage führen.

Ein hygroskopisch wirkender Sensor kann beispielsweise dazu dienen, einen Feuchtigkeitsanstieg im Innenraum 14 der Welle 10 festzustellen, der ein Indikator sein kann zum Hinweis, dass Kondensationswasser in der Welle vorhanden ist. Das Vorhandensein von Kondensationswasser kann auch als Indikator angesehen werden, dass eine minimale Leckage vorhanden ist. Auf jeden Fall ist die Kondensationswasserbildung in der Welle unerwünscht, da hierdurch korrosionsbedingte Schäden eher auftreten können. Bei den hier vorgesehenen Dimensionen des Fermenters mit einer Gesamtlänge zwischen üblicherweise 25 - 50 m Länge und einem Durchmesser zwischen 5 und 15 m weist entsprechend auch die Welle 10 einen Wellenkörper 11 auf mit einem Durchmesser, der zwischen 500 und 1500 mm betragen kann.

Bei diesen Dimensionen ist es selbstverständlich problemlos möglich, die Welle mit einer Einstiegsluke zu versehen. Durch die Einstiegsluke können entsprechende Revisionsarbeiten vorgenommen werden. Die Einstiegsluke muss selbstverständlich absolut dicht verschliessbar sein. Das Vorhandensein einer Einstiegsluke ist jedoch nicht zwingend. Bei einem möglichen Schaden kann selbstverständlich auch problemlos dieser durch Revisionsarbeiten von aussen her behoben werden. Durch die Weglassung einer Einstiegsluke werden Versteifungsprobleme in diesem Bereich vermieden, ebenso wie zusätzliche Leckagequellen.

Das Prinzip der Erfindung besteht, wie bereits erwähnt, im Wesentlichen darin, dass die Welle 10 als Hohlwelle gestaltet ist und diese einen entsprechenden Auftrieb besitzt. Dabei muss sichergestellt werden, dass man Überwachen kann, ob die Hohlwelle sich über eine Leckage mit Wasser füllt. In diesem Falle würde nämlich der Auftrieb wegfallen und die Welle würde sich entsprechend mehr und mehr durchbiegen, worauf unvermeidlich entsprechende Schäden auftreten würden. Um dies zu vermeiden wird erfindungsgemäss im Innenraum 14 der Welle ein vorgegebener Über- oder Unterdruck angelegt und dieser Druck mittels entsprechender Mittel überwacht.

### Bezugszeichenliste:

- 1: liegender Fermenter
- 2: Tank
- 3: Einlass
- 4: einlassseitige Stirnwand
- 5: Auslass
- 6: auslassseitige Stirnwand
- 7: Wellenlager
- 8: Wellenzapfen
- 9: biogene Abfälle
- 10: Welle
- 11: Wellenkörper
- 12: Rührarme
- 13: Schaufeln
- 14: Innenraum
- 15: Sensorüberwachungseinheit
- 16: Drucküberwachungseinheit
- 17: Druckleitung
- 18: Manometer
- 20: Fertigbauteil
- 21: Drucksensor
- 22: Temperatursensor oder hygroskopischer Sensor
- 23: Radiotransmitter
- 24: Übertragungsantenne
- 25: Mikrocomputerchip
- 26: Speiseeinheit

## Patentansprüche

1. Pfropfstrombetriebener liegender Fermenter (1) zur anaeroben Fermentation biogener Abfälle (9) mit einem Einlass (3) und einem Auslass (4) und einem Rührwerk, welches aus einer den Fermenter in seiner Längsrichtung durchquerenden Welle (10) besteht, an der eine Vielzahl von Rührarmen (12) angeordnet sind, wobei die Welle (10) im Bereich der Fermenterenden (4,6) gelagert ist, **dadurch gekennzeichnet, dass** mindestens die Welle (10) als geschlossenes Gas oder Luft gefülltes Hohlelement gestaltet ist und dessen Innenraum bezüglich des Druckes überwacht ist, um sicher zu stellen, dass deren Auftrieb im gefüllten Fermenter (1) die Durchbiegung der Welle mindestens annähernd kompensiert.

2. Pfropfstrombetriebener Fermenter nach Anspruch 1, **dadurch gekennzeichnet, dass** im Innenraum (14) ein überwachter Überdruck (P₁) anliegt.

3. Pfropfstrombetriebener Fermenter nach Anspruch 1, **dadurch gekennzeichnet, dass** im Innenraum (14) ein überwachter Unterdruck (P₁) anliegt.

4. Pfropfstrombetriebener Fermenter nach Anspruch 1, **dadurch gekennzeichnet, dass** im Innenraum ein druckmessendes Organ (15,16) angeordnet ist, dessen gemessener Wert überwacht ist.

5. Pfropfstrombetriebener Fermenter (1) nach Anspruch 1, **dadurch gekennzeichnet, dass** über die gesamte Länge der Welle (10) Rührarme (12) in regelmässigen Abständen und im gleichen Winkel am Umfang versetzt angeordnet sind.

6. Pfropfstrombetriebener Fermenter nach Anspruch 5, **dadurch gekennzeichnet, dass** jeweils zwei in Längsrichtung der Welle einander benachbarte Rührarme (12) um einen Winkel (α) zwischen 90° und 30°, bevorzugterweise um 45° versetzt angeordnet sind.

7. Pfropfstrombetriebener Fermenter nach Anspruch 1, **dadurch gekennzeichnet, dass** die Welle (10) mit Wellenzapfen versehen ist und die beiden endseitigen Wellenzapfen (8) die ein- und auslassseitigen Fermenterenden (4,6) durchsetzen und ausserhalb des Fermentertanks (2) gelagert sind.

8. Pfropfstrombetriebener Fermenter nach Anspruch 1, **dadurch gekennzeichnet, dass** ein den Wellenkörper (11) bildendes Rohr endseitig je einen Wellenzapfen (8) aufweist und mindestens endseitig über einen der Wellenzapfen angetrieben ist.

9. Pfropfstrombetriebener Fermenter nach Anspruch 1, **dadurch gekennzeichnet, dass** die Welle (10) mindestens antriebsseitig durch ein Fermenterende (4,6) durchgeführt ist und über einen darauf angebrachten Zahnkranz angetrieben ist.

10. Pfropfstrombetriebener Fermenter nach Anspruch 8, **dadurch gekennzeichnet, dass** in den Innenraum (14) der Welle (10) eine Druckleitung (17) führt, die durch einen der beiden Wellenzapfen (8) zu einem Messgerät geführt ist.

11. Pfropfstrombetriebener Fermenter nach Anspruch 1, **dadurch gekennzeichnet, dass** in den Innenraum (14) der Welle (10) eine in einer Sensorüberwachungseinheit (15) angeordnete mindestens eine Messsonde (21,22) vorhanden ist, wobei die Einheit (15) von ausserhalb des Fermenters erregbar ist und ein den gemessenen Daten entsprechendes Signal über einen Sender an einen ausserhalb des Fermenters angeordneten Empfänger liefert.

12. Pfropfstrombetriebener Fermenter nach Anspruch 1, **dadurch gekennzeichnet, dass** eine Änderung des im Innenraum (14) der Welle gemessenen Zustandes ein Signal an die Steueranlage des Fermenters liefert.

13. Pfropfstrombetriebener Fermenter nach Anspruch 1, **dadurch gekennzeichnet, dass** mindestens einige Rührarme (12) des Fermenters (1) als Hohlelemente gebildet sind, die einen erhöhten Auftrieb bewirken.

## Claims

1. Plug-flow operated horizontal fermenter (1) for the anaerobic fermentation of biogenic wastes (9), having an inlet (3) and an outlet (4) and a stirring mechanism consisting of a shaft (10) which crosses the fermenter in the longitudinal direction thereof and on which a plurality of stirring arms (12) are arranged, wherein the shaft (10) is mounted in the region of the ends (4, 6) of the fermenter, **characterised in that** at least the shaft (10) is designed as a closed, gas- or air-filled hollow element, the pressure in the interior of which is monitored in order to ensure that the buoyancy thereof in the filled fermenter (1) at least approximately compensates the bending of the shaft.

2. Plug-flow operated fermenter according to Claim 1, **characterised in that** a monitored overpressure (P₁) prevails in the interior (14).

3. Plug-flow operated fermenter according to Claim 1, **characterised in that** a monitored underpressure (P₁) prevails in the interior (14).

4. Plug-flow operated fermenter according to Claim 1, **characterised in that** a pressure-measuring element (15, 16) is arranged in the interior, the measured value of which element is monitored.

5. Plug-flow operated fermenter (1) according to Claim 1, **characterised in that** stirring arms (12) are arranged at regular intervals over the entire length of the shaft (10) and offset at the same angle on the circumference.

6. Plug-flow operated fermenter according to Claim 5, **characterised in that** in each case two stirring arms (12) which are adjacent to each other in the longitudinal direction of the shaft are arranged offset by an angle (α) between 90° and 30°, preferably by 45°.

7. Plug-flow operated fermenter according to Claim 1, **characterised in that** the shaft (10) is provided with shaft journals, and the two end shaft journals (8) penetrate the fermenter ends (4, 6) on the inlet and outlet sides and are mounted outside the fermenter tank (2).

8. Plug-flow operated fermenter according to Claim 1, **characterised in that** a tube forming the shaft body (11) has a shaft journal (8) at each end and is driven at least at the end by means of one of the shaft journals.

9. Plug-flow operated fermenter according to Claim 1, **characterised in that** the shaft (10) is guided through a fermenter end (4, 6) at least on the drive side and is driven by means of a ring gear attached thereto.

10. Plug-flow operated fermenter according to Claim 8, **characterised in that** a pressure line (17) leads into the interior (14) of the shaft (10), which pressure line is routed through one of the two shaft journals (8) to a measurement unit.

11. Plug-flow operated fermenter according to Claim 1, **characterised in that** at least one measurement probe (21, 22) which is arranged in a sensor monitoring unit (15) is present in the interior (14) of the shaft (10), wherein the unit (15) can be excited from outside the fermenter and supplies a signal corresponding to the measured data via a transmitter to a receiver arranged outside the fermenter.

12. Plug-flow operated fermenter according to Claim 1, **characterised in that** a change in the state measured in the interior (14) of the shaft supplies a signal to the control system of the fermenter.

13. Plug-flow operated fermenter according to Claim 1, **characterised in that** at least some stirring arms (12) of the fermenter (1) are formed as hollow elements which produce increased buoyancy.

## Revendications

1. Fermenteur entraîné par un écoulement piston (1) pour la fermentation anaérobies de déchets biogènes (9) comprenant une entrée (3) et une sortie (4) et un mélangeur qui est composé d'un arbre (10) traversant le fermenteur dans son sens longitudinal sur lequel une pluralité de bras mélangeurs (12) sont disposés, sachant que l'arbre (10) est disposé au niveau des extrémités du fermenteur (4, 6), **caractérisé en ce qu'**au moins l'arbre (10) est formé comme élément creux fermé rempli de gaz ou d'air et dont la pression est surveillée dans son espace intérieur afin de s'assurer que sa poussée verticale dans le fermenteur (1) rempli compense au moins approximativement le fléchissement de l'arbre.

2. Fermenteur entraîné par un écoulement piston selon la revendication 1, **caractérisé en ce qu'**une surpression surveillée (P₁) est présente dans l'espace intérieur (14).

3. Fermenteur entraîné par un écoulement piston selon la revendication 1, **caractérisé en ce qu'**une sous-pression surveillée (P₁) est présente dans l'espace intérieur (14).

4. Fermenteur entraîné par un écoulement piston selon la revendication 1, **caractérisé en ce qu'**un organe mesurant la pression (15, 16) dont la valeur mesurée est surveillée, est disposé dans l'espace intérieur.

5. Fermenteur entraîné par un écoulement piston (1) selon la revendication 1, **caractérisé en ce que** sur toute la longueur de l'arbre (10), des bras mélangeurs (12) sont disposés en étant décalés à distances régulières et avec le même angle sur le pourtour.

6. Fermenteur entraîné par un écoulement piston selon la revendication 5, **caractérisé en ce que** deux bras mélangeurs (12) respectifs voisins l'un de l'autre dans le sens longitudinal de l'arbre sont disposés en étant décalés d'un angle (α) entre 90° et 30°, de manière plus préférée de 45°.

7. Fermenteur entraîné par un écoulement piston selon la revendication 1, **caractérisé en ce que** l'arbre (10) est muni de tenons d'arbre et les deux tenons d'arbre (8) à l'extrémité traversent les extrémités du fermenteur (4, 6) côté entrée et côté sortie et sont disposés à l'extérieur de la cuve du fermenteur (2).

8. Fermenteur entraîné par un écoulement piston selon la revendication 1, **caractérisé en ce qu'**un tube formant le corps du fermenteur (11) présente un tenon d'arbre (8) à l'extrémité et est entraîné au moins à l'extrémité par l'un des tenons d'arbre.

9. Fermenteur entraîné par un écoulement piston selon la revendication 1, **caractérisé en ce que** l'arbre (10) est conduit au moins côté entraînement par une extrémité de fermenteur (4, 6) et est entraîné par une couronne dentée placée dessus.

10. Fermenteur entraîné par un écoulement piston selon la revendication 8, **caractérisé en ce qu'**une conduite sous pression (17) passe dans l'espace intérieur (14) de l'arbre (10), laquelle est dirigée vers un appareil de mesure par l'un des deux tenons d'arbre (8).

11. Fermenteur entraîné par un écoulement piston selon la revendication 1, **caractérisé en ce qu'**au moins une sonde de mesure (21, 22) placée dans une unité de surveillance par capteurs (15) est présente dans l'espace intérieur (14) de l'arbre (10), sachant que l'unité (15) peut être excitée depuis l'extérieur du fermenteur et fournit un signal correspondant aux données mesurées, par le biais d'un émetteur, à un récepteur placé à l'extérieur du fermenteur

12. Fermenteur entraîné par un écoulement piston selon la revendication 1, **caractérisé en ce qu'**une modification de l'état mesuré dans l'espace intérieur (14) de l'arbre fournit un signal à l'installation de commande du fermenteur.

13. Fermenteur entraîné par un écoulement piston selon la revendication 1, **caractérisé en ce qu'**au moins quelques bras mélangeurs (12) du fermenteur (1) sont formés comme des éléments creux qui ont pour effet une poussée verticale accrue.
